(19) Europäisches Patentamt
European Patent Office
Office européen des brevets

(11) Publication number : 0 614 895 A1

(12) EUROPEAN PATENT APPLICATION

(21) Application number : 94301628.7

(22) Date of filing : 08.03.94

(51) Int. Cl.$^5$ : **C07D 309/16,** C07D 493/20, C07D 497/20, C07B 53/00, C07B 57/00, // (C07D493/20, 319:00, 311:00, 311:00), (C07D497/20, 327:00, 311:00, 311:00)

(30) Priority : 08.03.93 GB 9304702
31.08.93 GB 9318038

(43) Date of publication of application :
14.09.94 Bulletin 94/37

(84) Designated Contracting States :
DE ES FR GB IT

(71) Applicant : Chiroscience Limited
Science Park
Milton
Cambridge CB4 4WE (GB)

(72) Inventor : Ley, Steven Victor
12 Wordsworth Grove
Cambridge CB3 9HH (GB)
Inventor : Woods, Martin
16 Phillips Avenue
Royston, Hertfordshire SG8 5ES (GB)

(74) Representative : Perry, Robert Edward
GILL JENNINGS & EVERY
Broadgate House
7 Eldon Street
London EC2M 7LH (GB)

(54) **Bis-dihydropyran derivatives and their use in chiral transformations.**

(57) A disubstituted bis-dihydropyran having C2 symmetry, in single enantiomer form can be used in an enantioselective reaction to form dispiroketal which are chiral templates.

EP 0 614 895 A1

**Field of the Invention**

This invention relates to dihydropyran derivatives and their use.

Background of the Invention

3,3',4,4'-tetrahydro-6,6'-bis-2H-pyran (abbreviated herein as "bis-DHP") is known. It selectively protects 1,2-diols, to form dispiroketals, and this protection is useful for carbohydrates; see Ley et al, Tet. Lett. (1992) 33: 4767-70, and Synthesis (1992) 52-54.

Summary of the Invention

According to a first aspect of the present invention, novel compounds are ring-substituted derivatives of bis-DHP, having C2 symmetry, in single enantiomer form. The formula (1) of 2,2'-disubstituted such compounds is shown in Scheme 1, and 5,5'-disubstituted compounds are shown by formula (1A). They are useful for the resolution of chiral 1,2-diols or related 1,2-difunctional compounds or for the desymmetrisation of prochiral 1,2-difunctional compounds, e.g. glycerol, or as catalysts.

In particular, a novel compound (1) may be reacted enantioselectively with a compound (2) to give a dispiroketal (3); see Scheme 1. The dispiroketals are themselves new. According to a further aspect of the invention, the dispiroketals may be used as a chiral template, or separated from a non-reacting enantiomer of racemic (2), and the bis-DHP unit may then be removed.

Description of the Invention

The present invention is based on the discovery that, when the bis-DHP contains substituents R, e.g. in the 2 and 2' positions, and the compounds are in substantially single enantiomeric form, they are highly selective in protecting diol functions of a specific chirality. Thus, treatment of compound (1) with a difunctional compound of formula R'-CH(XH)-CH(YH)-R" (2) will give specifically a dispiroketal of formula (3) (Scheme 1).

In the case of a compound (1) where R=Me, a synthesis is outlined as Scheme 2. The 2S,2S'-product is shown, but this process can be adapted for the preparation of any 2,2'- or other disubstituted-bis-DHP compound of the invention.

Examples of 2,2'-substituents R on the bis-DHP molcule are $C_{1-6}$ alkyl or $C_{6-10}$ aryl, e.g. methyl or phenyl or, more generally, higher alkyl, aryl, arylalkyl, etc. The nature of R is not critical, e.g. of up to 20 C atoms, but it is preferably inert. There may be other ring-substituents, provided that C2 symmetry and enantioselectivity to 1,2-difunctional compound (2) are retained. Thus R may be as shown in formula (1A), and these groups may be alternative or additional to 2,2'-substituents. The substituents shown in formula (1A) may have a function other than directing enantioselective reaction, however; such compounds may have utility, e.g. as resolving agents or auxiliaries or as asymmetric catalysis ligands, in which case R is chosen so that the compound (1A) is a bidentate ligand, e.g. R = -OH, $NR_2$ or $-PR_2$.

The compound (2) being protected could have additional substitution or functionality over that shown in Scheme 1. R' and/or R" will usually include a functional group, if it is desired to use the dispiroketal as a template; either may be a hydrocarbon radical or together may form a cyclic compound of up to 8 C atoms, and may optionally be substituted by OH, protected OH or another functional group. Examples of X and Y are -O-, -NH- and -S-, the requirement being that the functions allow formation of a dispiroketal of formula (3).

With reference to Scheme 1, in the case of chiral diol (X=Y=O and R'≠H or $CH_2OH$ when R"=H), the bis-DHP compound can act as a resolving agent by reacting preferentially only with the enantiomer that can form a compound of the relative stereochemistry shown in (3). Either enantiomer of (1) may be used as appropriate to select for either enantiomer of (2).

In the case where X=Y=O and R'=$CH_2OH$ (glycerol), the compound (2) is prochiral but protection with (1) provides desymmetrisation; compound (3) can then be used as a chiral C-3 synthon with benefits of stability in enantiomerically-pure form over those currently known (e.g. isopropylidine glycerol). Modification of the $CH_2OH$ group may be carried out before removal of the bis-DHP unit, to give a chiral product in single enantiomer form.

In an analogous reaction, this methodology was applied to a glycolate derivative. Thus, as shown in Scheme 3, reaction of glycolic acid with (S,S)-dimethyl-bisdihydropyran afforded a dispiroketal adduct (4) in good yield as a single diastereomer. Enolate formation, followed by quench with methyl iodide, gave the lactate derivative (5) as a 10:1 mixture of diastereomers. The selectivity of this initial alkylation is noteworthy. The

mixture of diastereomers (5) was further treated with base, then quenched with benzyl bromide, under the optimised conditions used for the natural lactate derivative, affording the fully substituted derivative (6). Deprotection afforded the ester (7). Hence, by choice of two electrophiles and the sequence in which they are incorporated, it is anticipated that the new chiral bis-DHP derivatives should allow access to a wide range of, say, $\alpha,\alpha$-disubstituted-$\alpha$-hydroxy acid derivatives in enantiopure form. This overcomes the dependence of other methods on the availability of a suitable chiral precursor acid.

The bis-DHP unit may be removed from a protected compound of formula (3) by equilibration with further material of formula (2). Thus, for example, in the case where X=Y=O and R'=$CH_2OH$, the primary hydroxy function can be derivatised with benzyl to give R'=$CH_2OCH_2Ph$, and then the specific benzyl-protected glycerol released by appropriate treatment of the derivative (3) with glycerol.

In a further embodiment of the invention, 2,2'-disubstituted-bis-DHP compounds of the invention can be reacted with $\alpha$-substituted carboxylic acids, where the $\alpha$-substituent is exemplified by hydroxyl or acylamino. Stereocontrolled alkylation reactions can be performed on the resulting dispiroketals, to give highly enantioselective syntheses of, for example, $\alpha$-hydroxy acids and $\alpha$-aminoacids (Scheme 1: X=O or NAc).

In this case, the chirality of the bis-DHP will determine that of the dispiroketal and thus the enantiomer formed of alkylated products. By this means, glycolic acid may be converted into one enantiomer of, for example, 2-hydroxypentanoic acid, and glycine may be converted into one enantiomer of, say, phenylalanine or $\alpha$-methylphenylalanine. If the $\alpha$-hydroxy or $\alpha$-aminoacid is chiral, then the stereochemistry of the dispiroketal would still be determined by the group R. For instance, it can be arranged that (S)- or (R)-phenylalanine is converted into (S)-$\alpha$-methylphenylalanine or, via epimerisation (with base) of the initial adduct, that (S)-phenylalanine may be converted into (R)-phenylalanine (Scheme 4).

Further, the chiral bis-dihydropyrans may be used to resolve such compounds as $\alpha$-amino or $\alpha$-hydroxyacids through preferential formation of one diastereomer. Scheme 5 illustrates this reaction for $\alpha$-hydroxyacids and a 2S,2'S-DHP derivative.

Scheme 6 gives examples of routes used to obtain useful compounds of formula (1A), as illustrated in the case of R = OH. In Scheme 6, the respective stating materials (8) and (9) are obtained by epoxidation of bis-DHP, followed by treatment with ethylene glycol, CSA and toluene, under reflux for 1 hour. Compound (8) is reacted (i) with triflic anhydride, pyridine, DCM at 0°C for 2h, and (ii) with $KO_2$, 18-C-6 catalyst, DMSO at RT-150°C. Compound (9) is reacted (i) with $Bu_2Sn(OMe)_2$ in benzene at reflux, (ii) with triflic anhydride, pyridine, DCM, and (iii) $KO_2$, 18-C-6, DMSO at RT.

Racemic compounds (1A) may be resolved by biocatalytic methods such as enzyme-mediated esterification or microbially-catalysed hydrolysis of a diester derivative, or chemically, e.g. by treatment of a phosphoric acid ester with a chiral amine.

The following Examples illustrate the invention. More specifically, Examples 1 and 2 illustrate the preparation of novel 2,2'-disubstituted-bis-DHP compounds of the invention; Examples 3 to 7 and 9A to 17 illustrate the preparation/modification of dispiroketals of the invention; and Examples 8 and 18 to 20 illustrate the deprotection of a dispiroketal, to give a single enantiomer product. "Dispoke" = bis-DHP.

With reference to Scheme 1, the following Table represents the different compounds used in the Examples.

| Example | X | Y | R | R' | R" |
|---|---|---|---|---|---|
| 3 | -O- | -O- | $-CH_3$ | $-CH_2OH$ | -H |
| 4 | -O- | -O- | $-CH_3$ | $-CH_2OCH_2Ph$ | -H |
| 5 | -O- | -O- | $-CH_3$ | -CHO | -H |
| 6 | -O- | -O- | -Ph | $-CH_2OH$ | -H |
| 7 | -O- | -O- | -Ph | $-CH_2OCH_2Ph$ | -H |
| 8 | -O- | -O- | -Ph | t-Bu | -H |
| 9 | -O- | -O- | -Ph | Sugar | |
| 10 | -O- | -O- | -Ph | $-(CH_2)_3CH_3$ | -H |
| 11 | -O- | -O- | -Ph | $-(CH_2)_4-$ | |
| 12 | -S- | -O- | -Me | $-CH_3$ | -H |
| 13 | -O- | -O- | -Ph | -Ph | -H |
| 14 | -O- | -O- | -Ph | $-(CH_2)_4OH$ | -H |
| 15 | -O- | -O- | -Ph | $-C(CH_3)_3$ | -H |
| 16 | -O- | -O- | -Ph | $-CH_3$ | $-CH_3$ |
| 17 | -O- | -O- | -Ph | $-CH_2CH_2OH$ | -H |
| 18 | -O- | -O- | -Ph | t-Bu | -H |
| 19 | -O- | -O- | -Ph | Bu | -H |
| 20 | -O- | -O- | -Ph | Bu | -H |

## EXAMPLE 1

Preparation of [2S, 2'S]-2,2'-Dimethyl-3,3',4,4'-tetrahydro-6,6'-bi-2H-pyran.

A solution of (S)-2-methyl-3,3',4,4'-tetrahydro-6-tri-n-butylstannylpyran in tetrahydrofuran is treated with n-butyl lithium at -78 degrees centigrade. Solid palladium chloride acetonitrile complex and cupric chloride are added and the mixture allowed to warm to room temperature. The reaction is quenched into ammonia/ammonium chloride and extracted with ether. Purification by column chromatography afford the title compound (50-75%).

[2S,2'S]-2,2'-dimethyl-3,3',4,4'-tetrahydro-6,6'-bi-2*H*-pyran $[\alpha]_D^{28}$-94.9 (c = 1, $CHCl_3$); $\delta_H$(270MHz, $CDCl_3$) 5.22 (2H, t, *J* 3.8, 5-H and 5'-H), 4.0-3.87 (2H, m, 2-H and 2'-H), 2.22-2.04 (4H, m, 3-$CH_2$ and 3'-$CH_2$), 1.88-1.77 (2H, m, 4-H and 4'-H), 1.61-1.45 (2H, m, 4-H and 4'-H), 1.32 (6H, d, *J* 6.4, 6-Me and 6'-Me); *m/z* 194 ($M^+$), 125, 97 ($C_6H_9O^+$), 91, 69, 57, 55; HRMS: Found ($M^+$, 194.1307), $C_{12}H_{18}O_2$ requires ($M^+$, 194.1307).

## EXAMPLE 2

**Preparation of [2*S*,2'*S*] 2,2'-Diphenyl-3,3',4,4'-tetrahydro-6,6'-bi-2*H*-pyran (27)**

BuLi (2.5 M in hexane; 5.0 mL, 12.59 mmol) was added dropwise to a solution of stannne (5.385 g, 11.9 mmol) in THF (100 mL) at -78 °C and stirred under argon for 30 minutes. A mixture of anhydrous copper (II) chloride (1.693 g 12.59 mmol) and palladium (II) chloride *bis* acetontrile complex (62 mg, 0.24 mmol) was added in one portion and the resultant brown slurry was vigorously stirred at -78 °C. After 3.25 h ammonia (20% 0.88 solution in sat. aq. $NH_4Cl$; 250mL) was added and the mixture warmed to r.t. The aqueous phase was extracted with ether (3 x 200mL) and the combined organic extracts were then washed sequentially with $H_2O$ (100mL) and brine (100 mL), dried ($MgSO_4$) and evaporated *in vacuo* to give a yellow oily solid. The oil was chromatographed on silica gel eluting with DCM/petrol (15-40% gradient elution) to give *diene* with no *meso* diene visible

by $^1$H nmr (appendix 2.2) as a white crystalline solid (1.358 g, 3.589 mmol, 60%) m.p. 116-124 °C, $[\alpha]_D^{25}$ = -119.9°(c = 1.00, $CHCl_3$); $\delta_H$ (200 MHz, $CDCl_3$), 7.40 (10H, m, Ph), 5.40 (2H, br.t, *J* 3.8, H-5, H-5'), 4.90 (2H,dd, *J* 10.0, 2.4, H-2, H-2'), 2.4-1.8 (8H, m, H-3, H-3', H-4, H-4').

**EXAMPLE 3**

Preparation of [2S, 6S, 7R, 9S, 14S]-2,9-Dimethyl-14-hydroxymethyl-1,8,13,16-tetraoxadispiro-[5.0.5.4]-hexadecane.

A solution of bis-dimethyl-DHP in toluene is treated with glycerol (excess) and camphorsulphonic acid (catalytic) and heated under reflux for 2 hours. The resulting yellow solution was cooled to room temp. and evaporated in vacuo to give a brown oil. Purification by column chromatography on silica gel afforded the dispoke adduct as a clear oil. (96%).

[2S, 6S, 7R, 9S, 14S]-2,9-dimethyl-14-hydroxymethyl-1,8,13,16-tetraoxadispiro-[5.0.5.4]hexadecane $[\alpha]_D^{22}$ - 48.7 (c = 1, $CHCl_3$); $\delta_H$(400MHz, $CDCl_3$), 4.06-4.00 (1H, m, 14-H), 3.80-3.71 (2H, m, 2-H and 9-H), 3.75 (1H, t, *J* 11.1, 15-$H_{ax}$), 3.65-3.54 (2H, m, 17-$CH_2$), 3.47 (1H, dd, *J* 11.2 and 2.95, 15-$H_{eq}$), 1.85 (1H, br, OH), 1.83-1.68 (4H, m, 5-$CH_2$ and 12-$CH_2$), 1.59-1.50 and 1.49-1.38 (8H, 2 x m, 3-$CH_2$, 4-$CH_2$, 10-$CH_2$ and 11-$CH_2$), 1.14 and 1.12 (6H, 2 x d, *J* 6.2, 2-Me and 9-Me); HRMS: Found ($MH^+$, 287.1858), $C_{15}H_{26}O_5$ requires ($MH^+$, 287.1858).

**EXAMPLE 4**

Preparation of [2S, 6S, 7R, 9S, 14S]-2,9-Dimethyl-14-benzyloxymethyl-1,8,13,16-tetraoxadispiro-[5.0.5.4]-hexadecane.

The benzyl ether of example 3 was prepared by treatment with sodium hydride and benzyl bromide in tetrahydrofuran.

**EXAMPLE 5**

Preparation of [2S, 6S, 7R, 9S, 14R]-2,9-Dimethyl-14-formyl-1,8,13,16-tetraoxadispiro-[5.0.5.4]-hexadecane.

The aldehyde was prepared by oxidation of the alcohol under standard conditions. [2S, 6S, 7R, 9S, 14R]-2,9-dimethyl-14-formyl-1,8,13,16-tetraoxadispiro[5.0.5.4]-hexadecane $[\alpha]_D^{28}$ - 57.5 (c = 1, $CHCl_3$); $\delta_H$(400MHz, $CDCl_3$) 9.67 (1H, s, CHO), 4.35 (1H, dd, *J* 10.6 and 4, 14H), 3.80-3.70 (2H, m, 2-H and 9-H), 3.73 (1H, t, *J* 11.2, 15-$H_{ax}$), 3.67 (1H, dd, *J* 11.2 and 4.0, 15-$H_{eq}$), 1.93-1.36 (12H, m, 3-$CH_2$, 4-$CH_2$, 5-$CH_2$, 10-$CH_2$, 11-$CH_2$ and 12-$CH_2$), 1.14 and 1.12 (6H, 2 x d, *J* 6.3, 2-Me and 9-Me).

**EXAMPLE 6**

**Preparation of [2*R*,6*R*,7*R*,9*R*,14*S*] 14-Hydroxymethyl-2,9-diphenyl-1,8,13,16-tetraoxadispiro[5.0.5.4]hexadecane**

A solution of CSA (50 mg, cat.), glycerol (867 mg, 9.42 mmol) and [2R,2'R] 2,2'-diphenyl-3,3',4,4'-tetrahydro-6,6'- bi-2H-pyran 26 (300 mg, 0.942 mmol) was heated under reflux for 16 h. The yellow solution was then cooled to r.t. and evaporated *in vacuo* to give a yellow foam which was purified by column chromatography on silica gel, eluting with ether/petrol (6:4), to furnish the alcohol as a white crystalline solid (277 mg, 0.678 mmol, 72%); m.p. 161-162 °C; $[\alpha]_{7D}^{27}$=-6.2° (c = 1.00 in $CHCl_3$); $\nu_{max}$ ($CHCl_3$)/$cm^{-1}$ 3446, 2943, 2869, 1493, 1453, 1215, 1173, 1043, 984, 754, 698; $\delta_H$ (200 MHz , $CDCl_3$) 7.37 (10H, m, Ph), 4.74 (1H, dd, *J* 11.6, 2.2, H-2, H-9), 4.36 (1H, m, H-14), 3.82 (1H, t, *J* 11.0, H-$15_{ax}$), 3.64 (1H, dd: 11.7, 3.7, H-$1'_A$), 3.35 (1H, dd, *J* 11.7, 5.7, H-$1'_B$), 3.46 (1H, dd, *J* 11.0, 2.8, H-$15_{eq}$), 2.2-1.5 (12H, m, H-3, H-4, H-5, H-10, H-11, H-12); *m/z* 410($M^+$), 392, 319 ($26H^+$), 250, 235, 177, 130, 117; Found ($M^+$) 410.2102. $C_{25}H_{30}O_5$ requires 410.2093.

**EXAMPLE 7**

**Preparation of [2*R*,6*R*,7*R*,9*R*,14*S*] 14-Benzyloxymethyl-2,9-diphenyl-1,8,13,16-tetraoxadispiro[5.0.5.4]hexadecane**

Sodium hydride (60% dispersion in mineral oil; 63 mg, 1.59 mmol) was added in one portion to a solution of alcohol 93 (217 mg, 0.529 mmol) in THF ( 10 mL) at 0°C. The resultant suspension was warmed to r.t. (approx. 30 minutes), benzyl bromide injected (190 μL, 1.6 mmol) and the mixture further stirred for 16 h. The reaction was quenched by pouring into $H_2O$ (10 mL) and the aqueous phase extracted with ether (3 x 20 mL). The combined organic extracts were washed with brine (10 mL), dried ($MgSO_4$) and evaporated *in vacuo* to give the crude *benzyl ether* as an oil which crystallised on the addition of ether/petrol (1:9) (260 mg, 0.503 mmol, 98%); m.p. 164-167°C; $[\alpha]_D^{26}$ = +21.2 ° (c = 0.25 in $CHCl_3$); $\nu_{max}$ ($CHCl_3$)/$cm^{-1}$ 2944, 2867, 1603, 1495, 1453, 1366, 1274, 1203, 1174, 1099, 1043, 984, 909, 746, 698; $\delta_H$ (200 MHz , $CDCl_3$) 7.30 (15H, m, Ph), 4.76 (2H, dt, *J* 11.7, 2.6, H-2, H-9), 4.55 (2H, s, PhC<u>H</u>$_2$O), 4.21 (1H, m, H-14), 3.77 (1H, t, *J* 11-0, H-$15_{ax}$), 3.61-3.40 (3H, m, H-1', H-$15_{eq}$), 2.1-1.4 (12H, m, H-3, H-4, H-5, H-10, H-11, H-12); *m/z* (EI) 409 (M-Bu), 390, 375, 319 (26H+), 292, 135, 117, 73; Found 409.2007 (M-Bu). $C_{25}H_{29}O_5$ requires 409.2015.

**EXAMPLE 8**

**Preparation of [2*R*] 1-Benzyloxy-2,3-dihydroxypropane (99) and (2*R*,6*R*,7*R*,9*R*,14*S*] 14-*tert*-butyl-2,9-diphenyl-1,8,13,16-tetraoxadispiro[5.0.5.4]hexadecane**

A solution of the benzyl ether derivative (105 mg, 0.210 mmol), 3,3-dimethyl-1,2-butanediol (500 mg, 4.2 mmol) and CSA (24 mg, 0.105 mmol) in toluene (1 mL) was heated at reflux for 7 h. The yellow solution was chromatographed, without evaporation of toluene, on silica gel eluting with ethyl acetate/petrol 10-100%. This gave in order of elution, tbutyl adduct as a white waxy solid (85 mg, 0195 mmol, 93%) with data consistent with that of 102 described below, and benzyl ether 99 as a colourless oil (30 mg, 0.164 mmol, 78%), $[\alpha]_D^{25}$ = 0° (c = 1.00 in $CHCl_3$); $\nu_{max}$ (film)/$cm^{-1}$ 3396, 2867, 1647, 1453, 1362, 1207, 1074, 739, 698; $\delta_H$ (200 MHz, $CDCl_3$) 7.33 (5H, m, Ph), 4.53 (2H, s, PhC<u>H</u>$_2$O), 3.87 (1H, m, H-2), 3.65 (2H,m , H-3 (on $D_2O$ shake collapses to AB system 3.67 (1H, dd, *J* 11.5, 3.8, H-$3_A$) and 3.57 (1H, dd, *J* 11.5, 5.7, H-$3_B$)), 3.52 (2H, m, H-1), 2.90 (1H, br.s, OH), 2.47 (1H, br.s, OH); *m/z* (EI) 182 ($M^+$), 121 ($PhCH_2OCH_2^+$), 91 ($C_7H_7^+$), 77 ($Ph^+$); Found ($M^+$) 182.0944. $C_{10}H_{14}O_3$ requires 182.0943.

**EXAMPLE 9A**

**Preparation of [2'*R*,2"*R*,6'*R*,6"*R*] 2-*O*,5-*O*-Dibenzoyl-3-*O*,4-*O*-(6,6'-diphenyloctahydro-2,2'-bipyran-2,2'-diyl)-*myo*-inositol**

A solution of 2-*O*,4-*O*-dibenzoyl-*myo*-inositol (136 mg, 0.35 mmol), [2*R*,2'*R*] 2,2'-diphenyl-3,3',4,4'-tetrahydro-6,6'-bi-2*H*-pyran (167 mg, 0.524 mmol) and CSA (20 mg, cat.) in $CHCl_3$ (3 mL) was heated under reflux for 16 h.

The solution was cooled to r.t, evaporated *in vacuo* and chromatographed on silica gel eluting with ether/petrol (8:2) to give the diol as an amorphous white solid (95 mg, 0.134 mmol, 38 %); m.p. 195-207°C; $[\alpha]_D^{27}$ = + 46.5° (c = 1.00, $CHCl_3$); $^{\nu}$max ($CHCl_3$)/$cm^{-1}$ 3422, 2944, 1729, 1602, 1450, 1270, 1177, 1096, 1049, 1027, 968, 698; $\delta_H$ (200 MHz, $CDCl_3$) 8.20 (2H, m, *o*-OCO<u>Ph</u>), 7.80 (2H, m, *o*-OCO<u>Ph</u>), 7.6-7.05 (16H, m, *m*- and *p*-OCO<u>Ph</u>, Ph), 5.75 (1H, br.t, *J* 2.5, H-2), 5.26 (1H, t, *J* 9.7, H-5), 4.73 (2H, br.d, *J* 11.4, H-6', H-6"), 4.43 (1H, t, *J* 10.1, H-4), 3.96 (2H, m, H-6, H-3), 3.80 (1H, br.dd, *J* 10.0, 2.5, H-1), 3.10 (2H, br.s, (C-1)O<u>H</u>,(C-6)O<u>H</u>), 2.05-1.3 (12H, H-3', H-4', H-5', H-3", H-4", H-5"); $\delta_C$(50.3MHz, $CDCl_3$) 166.9, 166.6, 143.2, 142.9, 133.3, 132.8, 129.82, 129.77, 128.4, 128.3, 127.2, 127.0, 125.7, 125.5, 98.1, 97.8, 73.9, 73.0, 72.1, 71.8, 71.5, 66.5, 66.1, 33.6, 33.2, 28.0, 27.8, 18.8, 18.6; *m/z* (FAB) 729 ($MNa^+$), 707 ($MH^+$), 689 (M-OH), 585, 530, 391, 371, 319 ($26H^+$); Found ($MH^+$) 707.2807. $C_{42}H_{43}O_{10}$ requires 707.2855.

**EXAMPLE 9B**

**Preparation of [2'*S*,2"*S*,6'*S*,6"*S*] 2-*O*,5-O-Dibenzoyl-1-*O*,6-*O*-(6,6'-diphenyloctabydro-2,2'-bipyran-2,2'-diyl)-*myo*-inositol**

A solution of 2-*O*,4-*O*-dibenzoyl-*myo*-inositol (204 mg, 0.525 mmol), -[2*S*,2'*S*] 2,2'-diphenyl-3,3',4,4'-tetrahydro-6,6'-bi-2*H*-pyran (172 mg, 0.540 mmol) and CSA (30 mg, cat.) in $CHCl_3$ (5 mL) was heated under reflux for 16 h. The solution was cooled to r.t, evaporated *in vacuo* and chromatographed on silica gel eluting with ether/petrol (8:2) to give the diol as an amorphous white solid (169 mg, 0.239 mmol, 46%); m.p. 198-202°C; $[\alpha]_D^{27}$ = -51.6° (c = 1.00, $CHCl_3$); with identical spectra data to enantiomeric Example 6.

**EXAMPLE 10A**

**Preparation of [2*S*,6*S*,7*R*,9*S*,14*R*] 14.Butyl-2,9-diphenyl-1,8,13,16- tetraoxadispiro[5.0.5.4]hexadecane**

CSA (10mg, cat), hexane-1,2-diol (41 µL, 0.32 mmol) and [2S,2'S] 2,2'-diphenyl-3,3'4,4'-tetrahydro-6,6'-bi-2H-(500 µL) under argon for 24 h. The reaction mixture was then chromatographed on silica gel eluting with ether/petrol (5: 95)) to give the dispoke *derivative* as a white crystalline solid (64 mg, 0.147 mmol, 93%); m.p.

101-104°C; $[\alpha]_D^{25}$ = +2.5° (c = 1.00 in $CHCl_3$); $\nu_{max}$ ($CHCl_3$)/$cm^{-1}$ 3054, 2034, 2870, 1453, 1202, 1171, 1062, 1044, 980, 739, 700; $\delta_H$ (200 MHz , $CDCl_3$) 7.40 (10H, m, Ph), 4.75 (1H, dd, *J* 11.5, 2.3, H-2 (or H-9)), 4.71 (1H, dd, *J* 11.5, 2.3, H-9 or H-2), 3.91 (1H, m, H-14), 3.61 (1H, t, *J* 10.8, H-$15_{ax}$), 3.41 (1H, dd, *J* 10.8, 2.8, H-$15_{eq}$), 2.1-1.3 (18H, m, H-1', H-2', H-3', H-3, H-4, H-5, H-10, H-11, H-12), 0.90 (3H, t, *J* 7.2, H-4'); *m/z* (EI) 436 ($m^+$), 319 ($27H^+$), 276, 260, 177, 130, 117, 104 ($PbCHCH_2$); Found ($M^+$) 436.2596. $C_{28}H_{36}O_4$ requires 436.2613; Found:C, 77.22; H, 8.44. $C_{28}H_{36}O_4$ requires C, 77.03; H, 8.31%.

**EXAMPLE 10B**

**Preparation of [2*R*,6*R*,7*S*,9*R*,14*S*] 14-Butyl-2,9-diphenyl-1,8,13,16- tetraoxadispiro[5.0.5.4]hexadecane**

CSA (20 mg, cat), hexane-1,2-diol (91 µL, 0.73 mmol) and [2*R*,2'*R*] 2,2'-diphenyl-3,3',4,4'-tetrahydro-6,6'-bi-2*H*-pyran 27 (106 mg, 0.332 mmol) were heated at reflux in toluene (1 mL) under argon for 24 h. The reaction mixture was then chromatographed on silica gel eluting with ether/petrol (5:95) to give *dispoke derivative* 119 as a white crystalline solid (130 mg, 0.298 mmol, 90%); m.p. 101-104°; $[\alpha]_D^{25}$ = -1.9°; with spectral data consistent with that of Example 10A.

**EXAMPLE 11A**

**Preparation of [1*R*,2*R*,2'*S*,2"S,6'S,6"S] 1-*O*,2-*O*-(6',6"-diphenyloctahydro-2',2"-bipyran-2',2"-diyl)cyclohexane-1,2-diol**

CSA (10 mg, cat.), *trans*-cyclohexane-1,2-diol (15 mg, 0.132 mmol) and [2*S*,2'*S*] 2,2'-diphenyl-3,3',4,4'-tetrahydro-6,6'-bi-2*H*-pyran (20 mg, 0.0628 mmol) were heated at reflux in toluene (300 µL) under argon for 8 h. The reaction mixture was then chromatographed on silica gel eluting with ether/petrol (5:95) to give the *dispoke derivative* as a white crystalline solid (22 mg, 0.0506 mmol, 80%); m.p. 165-167 °C; $[\alpha]_D^{21}$ = -18.3° (c = 1.00 in $CHCl_3$); ,$\nu_{max}$ ($CHCl_3$)/$cm^{-1}$ 2940, 1452, 1216, 1169, 1065, 1042, 986, 971, 755, 699, 667; $\delta_H$ (200 MHz , $CDCl_3$) 7.40 (10H, m, Ph), 4.75 (2H, dd, *J* 11.6, 2.3, H-6', H-6"), 3.55 (2H, m, H-1, H-2), 2.1- 1.3 (20H, m, H-3, H-4, H-5, H-6, H-3', H-4', H-5', H-3", H-4", H-5"); *m/z* (EI) 434 ($M^+$), 258, 177, 160, 130, 117, 104 ($PhCHCH_2^+$); Found ($M^+$) 434.2461. $C_{19}H_{23}O_6Si$ requires 434.2457; Found: C, 77.48; H, 8.17. $C_{28}H_{34}O_4$ requires C, 77.39; H, 7.89).

**EXAMPLE 11B**

**Preparation of [1*S*,2*S*,2'*R*,2"*R*,6'*R*,6"*R*] 1-*O*,2-*O*-(6',6"-diphenyloctahydro-2',2"-bipyran-2',2"-diyl)cyclohexane-1,2-diol**

CSA (30 mg, cat.), *trans*-cyclohexane-1,2-diol (15 mg, 0.132 mmol) and [2*R*,2'*R*] 2,2'-diphenyl-3,3',4,4'-tetrahydro-6,6'-bi-2*H*-pyran (100 mg, 0.314 mmol) were heated at reflux in toluene (1 mL) under argon for 16 h. The reaction mixture was then chromatographed on silica gel eluting with ether/petrol (5:95) to give the *dispoke* derivative as a white crystalline solid (97 mg, 0.223 mmol, 71%); m.p. 167°C; $[\alpha]_D^{27}$ = +13.5° (c = 1.00 in $CHCl_3$); with spectra data consistent with that of Example 11A.

**EXAMPLE 12**

**Preparation of [2*S*,6*S*,7*R*,9*S*,14*R*] 2,9-diphenyl-14-methyl-16-thia-1,8,13-trioxadispiro[5.0.5.4]hexadecane**

CSA (10 mg, cat), 1-mercaptopropan-2-ol (12 µL, 0.132mmol) and [2*S*,2'*S*] 2,2'-diphenyl-3,3',4,4'-tetrahydro-6,6'-bi-2*H*-pyran (20 mg, 0.0628 mmol) were heated at a reflux in toluene (300 µL) under argon for 16 h. The reaction mixture was then chromatographed on silica gel eluting with ether/petrol (5:95) to give the dispoke *derivative* as a white solid (24.7 mg, 0.0602mmol, 96%); m.p. 160-163 °C; $[\alpha]_D^{25}$ = +65.6° (c = 1.00); $\nu_{max}$ ($CHCl_3$)/$cm^{-1}$ 2932, 1450, 1210, 1097, 1046, 1008, 744, 696; $\delta_H$ (200 MHz , $CDCl_3$) 7.35 (10H, m, Ph), 5.27 (1H, dd, *J* 11.6, 2.1, H-2 (or H-9), 4.70 (1H, dd, *J* 11.6, 2.2, H-9 (or H-2)), 4.14 (1H, ddq, 10.7, 1.8, 6.4, H-14), 2.68 (1H, dd, 12.8, 10.8, H-$15_{ax}$), 2.06 (1H, dd, *J* 13.2, 1.8, H-$15_{eq}$), 2.04-1.3 (12H, m, H-3, H-4, H-5, H-10, H-11, H-12), 1.19 (3H, d, *J* 6.4, Me); *m/z* (EI) 410 ($M^+$), 368, 319 ($27H^+$), 234, 192, 177, 160, 117, 104 ($PhCHCH_2^+$), 91, 77 ($Ph^+$); Found ($M^+$) 410.1914. $C_{25}H_{30}O_3S$ requires 410.1916.

**EXAMPLE 13**

**Preparation of [2*S*,6*S*,7*R*,9*S*,14*R*] 2,9,14-Triphenyl-1,8,13,16-tetraoxadispiro[5.0.5.4]hexadecane**

CSA (10mg, cat), styrenediol (26 mg, 0.188 mmol) and [2*S*,2'*S*] 2,2'-diphenyl-3,3',4,4'-tetrahydro-6,6'-bi-2H-pyran (30 mg, 0.094 mmol) were heated at reflux in toluene (300 µL) under argon for 24 h. The reaction mixture was then chromatographed on silica gel eluting with ether/petrol (5:95) to give the *dispoke derivative* as a colourless oil 15 mg, 0.033 mmol, 35%); $[\alpha]_D^{25}$ = -44.3° (c = 1.00 in $CHCl_3$); $\nu_{max}$ ($CHCl_3$)/$cm^{-1}$ 2944, 1604, 1494, 1452, 1366, 1272, 1209, 1170, 1101, 1040, 983, 918, 868, 753, 698; $\delta_H$ (200 MHz , $CDCl_3$) 7.35 (15H, m, Ph), 5.02 (1H, dd, *J* 10.6, 3.1, H-14), 4.78 (2H, br.d, *J* 11.6, H-2, H-9), 3.82 (1H, t, *J* 11.0, H-$15_{ax}$), 3.60 (1H, dd, 11.2, 3.2, H-$15_{eq}$), 2.3-1.5 (12H, m, H-3, H-4, H-5, H-10, H-11, H-12); *m/z* (EI) 456 ($M^+$), 280, 176, 160, 117, 104, 91 ($C_7H_7^+$); Found ($M^+$) 456.2274. $C_{30}H_{32}O_4$ requires 456.2300.

## EXAMPLE 14A

**Preparation of [2*S*,6*S*,7*R*,9*S*,14*R*] 2,9-diphenyl- 14-(4-hydroxybut-1- yl)-1,8,13,16-tetraoxadispiro[5.0.5.4]hexadecane**

CSA (20mg, cat), hexane-1,2,6-triol (40 µL, mmol) and [2**S**,2'**S**] 2,2'-diphenyl-3,3',4,4'-tetrahydro-6,6'-bi-2H-pyran (30 mg, 0.0942 mmol) were heated at reflux in toluene (500 µL) under argon for 18 h. The reaction mixture was then chromatographed on silica gel eluting with ether/petrol (6:4) to give the *dispoke derivative* as a viscous oil (20 mg, 0.044 mmol, 47%); $[\alpha]_D^{25}$ = +5.6° (c = 0.43 in $CHCl_3$); $\nu_{max}$ ($CHCl_3$)/$cm^{-1}$ 3146, 2938, 2866, 1452, 1365, 1273, 1202, 1170, 1044, 983, 729, 698 ;$\delta_H$ (200 MHz , $CDCl_3$) 7.35 (10H, M, Ph), 4.72 (1H, dd, *J* 11.4, 2.3, H-2 (or H-9)), 4.69 (1H, dd, *J* 11.3, 2.3, H-9 or H-2), 3.79 (1H, m, H-14), 3.64 (2H, m, H-1'), 3.61 (1H, t, *J* 10.7, H-$15_{ax}$), 3.40 (1H, dd, *J* 11.0, 2.8, H-$15_{eq}$), 2.1-1.3 (18H, m, H-2', H-3', H-4', H-3, H-4, H-5, H-10, H-11, H-12); *m/z* (EI) 452, 434, 419, 395, 319, 276, 177, 160, 130, 117; Found ($M^+$) 452.2551. $C_{28}H_{36}O_5$ requires 452.2563.

## EXAMPLE 14B

**Preparation of [2*R*,6*R*,7*S*,9*R*,14*S*] 2,9-diphenyl-14-(4-hydroxybut-1- yl)-1,8,13,16-tetraoxadispiro[5.0.5.4]hexadecane**

CSA (10 mg, cat), hexane-1,2,6-triol (23 µL, 0.188mmol) and [2*R*,2'*R*] 2,2'-diphenyl-3,3',4,4'-tetrahydro-6,6'-bi-2H-pyran (30 mg, 0.0942 mmol) were heated at reflux in toluene (500 µL) under argon for 18 h. The reaction mixture was then chromatographed on silica gel eluting with ether/petrol (6:4) to give the *dispoke derivative* as a viscous oil (18 mg, 0.040 mmol, 42%); $[\alpha]_D^{25}$ = - 4.2° (c = 1.00 in $CHCl_3$); with spectra data consistent with that of Example 14A above.

## EXAMPLE 15

**Preparation of [2*R*,6*R*,7*R*,9*R*,14*S*] 14-*tert*-butyl-2,9-diphenyl-1,8,13,16-tetraoxadispiro[5.0.5.4]hexadecane**

CSA (10mg, cat), 3,3-dimethylbutane-1,2-diol (85% tech grade; 37 mg, 0.266 mmol) and [2*R*,2'*R*] 2,2'-diphenyl-3,3',4,4'-tetrahydro-6,6'-bi-2H-pyran (40 mg, 0.127 mmol) were heated at reflux in toluene (500 µL) under argon for 18 h. The reaction mixture was then chromatographed on silica gel eluting with ether/petrol (5:95) to give the *dispoke derivative* as a white solid (49.5 mg, 0.113 mmol, 90%); m.p. 101-105 °C; $[\alpha]_D^{26}$ = - 19.8° (c = 1.00 in $CHCl_3$); $\nu_{max}$ ($CHCl_3$)/$cm^{-1}$ 2948, 2868, 1493, 1478, 1451, 1365, 1202, 1170, 1042, 985, 973, 748, 697; $\delta_H$ (200 MHz, $CDCl_3$) 7.36 (10H, m, Ph), 4.75 (1H, dd, *J* 11.4, 23, H-2 (or H-9)), 4.73 (1H, dd *J* 11.7, 2.5, H-9 (or H-2)), 3.78 (1H, t, *J* 10.3, H-$15_{ax}$), 3.62 (1H, dd, *J* 10.6, 2.2, H-14), 3.50 (1H, dd, *J* 10.0, 2.2, H-$15_{eq}$), 2.1-1.4 m, H-3, H-4, H-5, H-10, H-11, H-12), 0.93 (3H, s, $^tBu$); *m/z* (EI) 436 ($M^+$), 319 ($26H^+$), 260, 177, 130, 117, 104 ($PhCHCH_2$); Found ($M^+$) 436.2607. $C_{35}H_{28}O_4$ requires 436.2613.

## EXAMPLE 16A

**Preparation of [2*R*,6*R*,7*R*,9*R*,14*S*,15*S*] 14,15-dimethyl-2,9-diphenyl-1,8,13,16-tetraoxadispiro[5.0.5.4]hexadecane**

CSA (10mg, cat), butane-1,2-diol (racemic $C_2$; 30 mg, 0.33 mmol) and [2*R*,2'*R*] 2,2'-diphenyl-3,3',4,4'-tetrahydro-6,6'-bi-2H-pyran (50 mg, 0.157 mmol) were heated at reflux in toluene (500 µL) under argon for 48 h. The reaction mixture was then chromatographed on silica gel eluting with ether/petrol (5:95) to give the *dispoke derivative* as a white solid (47 mg, 0.234 mmol, 71%); m.p. 114-117 °C; $[\alpha]_D^{26}$ = +27.6° (c = 1.00 in $CHCl_3$); $\nu_{max}$ ($CHCl_3$)/$cm^{-1}$ 2936,1493, 1380, 1202, 1180, 1070, 1047, 991, 909, 733, 698; $\delta_H$ (200 MHz, $CDCl_3$) 7.40 (10H, m, Ph), 4.71 (1H, dd, *J* 11.5, 2.3, H-2, H-9), 3.66 (2H, m, H-14, H-15), 2.1-1.3 (12H, m ,H-3, H-4, H-5, H-10,

H-11, H-12), 1.07 (6H, s, (C-14)Me, (C-15)Me); *m/z* (EI) 408 ($M^+$), 353, 319 ($26H^+$), 274, 232, 177, 130, 117, 104 ($PhCHCH_2$); Found ($M^+$) 408.2305. $C_{26}H_{32}O_4$ requires 408.2300.

**EXAMPLE 16B**

**Preparation of [2*R*,6*R*,7*R*,9*R*,14*S*,15*R*] 14,15-dimethyl-2,9-diphenyl 1,8,13,16-tetraoxadispiro[5.0.5.4]hexadecane and [2*R*,6*R*,7*R*,9*R*,14*S*,15*S*] 14,15-dimethyl-2,9-diphenyl-1,8,13,16-tetraoxadispiro[5.0.5.4]hexadecane**

CSA (10mg, cat), butane-1,2-diol (mix of $C_2$:meso, ex. Aldrich; 187 μL, 2.07 mmol) and [2*R*,2'*R*] 2,2'-diphenyl-3,3',4,4'- tetrahydro-6,6'-bi-2H-pyran (200 mg, 0.628 mmol) were heated at reflux in toluene (3 mL) under argon for 20 h. The reaction mixture was then chromatographed on silica gel eluting with ether/petrol (5:95) to give, in order of elution, the diequatorial the *dispoke derivative* as a crystalline solid (38 mg, 0.093 mmol, 15%) with data consistent with that of Example 16A above, and the *dispoke derivative* (174 mg, 0.426 mmol, 68%); m.p. 171-172 °C; $[\alpha]_D^{24}$= -3.6 ° (c = 0.50 in $CHCl_3$); $\nu_{max}$ ($CHCl_3$)/$cm^{-1}$ 2940, 1450, 1381, 1202, 1166, 1081, 1045, 978, 756, 697; $\delta_H$ (200 MHz , $CDCl_3$) 7.38 (10H, m, Ph), 5.00 (1H, dd, *J* 11.5, 2.2, H-2 (or H-9)), 4.70 (1H, dd, *J* 11.5, 2.2, H-9 (or H-2)), 4.33 (1H, dq, *J* 6.7, 3.6, H-15), 3.75 (1H, dq, *J* 7.0, 3.6, H-14), 2.2-1.3 (12H, m, H-3, H-4, H-5, H-10, H-11, H-12), 1.31 (3H, d, *J* 7.0, (C-14)Me), 1.08 (3H, d, *J* 6.7, (C-15)Me); *m/z* (EI) 408 ($M^+$), 353, 319 ($26H^+$), 274, 232, 177, 130, 117, 104 ($PhCHCH_2$); Found: C, 76.43; H, 7.90. $C_{26}H_{32}O_4$ requires C, 76.44; H, 7.90).

**EXAMPLE 17**

**Preparation of [2*R*,6*R*,7*R*,9*R*,14*S*] 14-(1-Hydroxyeth-2-yl)-2,9- diphenyl-1,8,13,16-tetraoxadispiro[5.0.5.4]hexadecane**

CSA (20mg, cat), butane-1,2,4-triol (34 μL, 0.377 mmol) and [2*R*,2'*R*] 2,2'-diphenyl-3,3',4,4'-tetrahydro-6,6'-bi-2H-pyran (40 mg, 0.127 mmol) were heated at reflux in toluene (500 μL) under argon for 24 h. The reaction mixture was then chromatographed on silica gel eluting with ether/petrol (60:40) to give the *dispoke derivative* as a colourless foam (32.7 mg, 0.077 mmol, 62%); m.p. 156-158°C; $[\alpha]_D^{27}$ = -15.4° (c = 1.00 in $CHCl_3$); $\nu_{max}$ ($CHCl_3$)/$cm^{-1}$ 3447, 2927, 2854, 1719, 1493, 1453, 1214, 1172, 1044, 981, 755, 698; $\delta_H$ (200 MHz , $CDCl_3$) 7.37 (10H, m, Ph), 4.73 (2H, dd, *J* 11.6, 1.8, H-2, H-9), 4.21 (1H, m, H-14), 3.85 (2H, br.s, H1'[$D_2O$ shake, d, *J* 5.5]), 3.71 (1H, t, *J* 10.9, H-$15_{ax}$), 3.39 (1H, dd, *J* 11.1, 2.8), 2.1-1.5 (14H, m, H-2', H-3, H-4, H-5, H-10, H-11, H-12); *m/z* (EI) 424 ($M^+$), 406 (M-OH), 319 (26H+), 248, 177, 130, 117, 104 ($PhCHCH_2$); Found ($M^+$) 424.2256. $C_{26}H_{32}O_5$ requires 424.2250.

**EXAMPLE 18**

**Treatment of [2*S*,6*S*,7*R*,9*S*,14*R*) 14-Butyl-2,9-diphenyl-1,8,13,16-tetraozadispiro[5.0.5.4]hexadecane with Ferric Chloride.**

Anhydrous ferric chloride (96 mg, 0.596 mmol) was added in one portion to a solution of dispoke adduct in DCM (130 mg, 0.298 mmol). The brown solution was stirred for 1 h and quenched with $H_2O$ (100 μL). The organic solvents were removed *in vacuo* and the subsequent wet yellow oil was lyophilised. The crude hexane diol was chromatographed on silica gel, eluting ether. The yellow oil obtained (14 mg, 0.118 mmol, 40%) was derivatised to the 1,2-ditrifluoroacetate and its structure confirmed by g.c. analysis.

**EXAMPLE 19**

**Treatment of [2*R*,6*R*,7*S*,9*R*,14*S*] 14-Butyl-2,9-diphenyl-1,8,13,16-tetraoxadispiro[5.0.5.4]hexadecane with Ferric Chloride.**

Anhydrous ferric chloride (65 mg, 0.404 mmol) was added in one portion to a solution of dispoke adduct (88 mg, 0.202 mmol). The brown solution was stirred for 16 h and quenched with $H_2O$ (100μL). The organic solvents were removed *in vacuo* and the subsequent wet yellow oil was lyophilysed. The crude hexane diol was chromatographed on silica gel, eluting with ether. The yellow oil obtained (16 mg, 0.135 mmol, 67%) was derivatised to the 1,2-ditrifluoroacetate and its structure confirmed by g.c. analysis.

**EXAMPLE 20**

**Treatment of [2*R*,6*R*,7*S*,9*R*,14*S*] 14-Butyl-2,9-diphenyl-1,8,13,16-tetraoxadispiro[5.0.5.4]hexadecane of**

**with Tin (IV) Chloride.**

Anhydrous tin (IV) chloride (1M in DCM; 24 µL, 0.243 mmol) was added in one portion to a solution of dispoke adduct 117 (53 mg, 0.121 mmol). The brown solution was stirred for 17 h and quenched with $H_2O$ (1 mL) and KF (spat. end). The organic solvents were extracted with DCM (3 x 10 mL). The combined organic extracts were dried ($MgSO_4$) and the solvent removed *in vacuo*. The crude diol was chromatographed on silica gel eluting with ether to give hexane diol (11 mg, 0.96 mmol, 79%). The yellow oil obtained was derivatised to the 1,2-ditrifluoroactate and its structure confirmed by g.c. analysis.

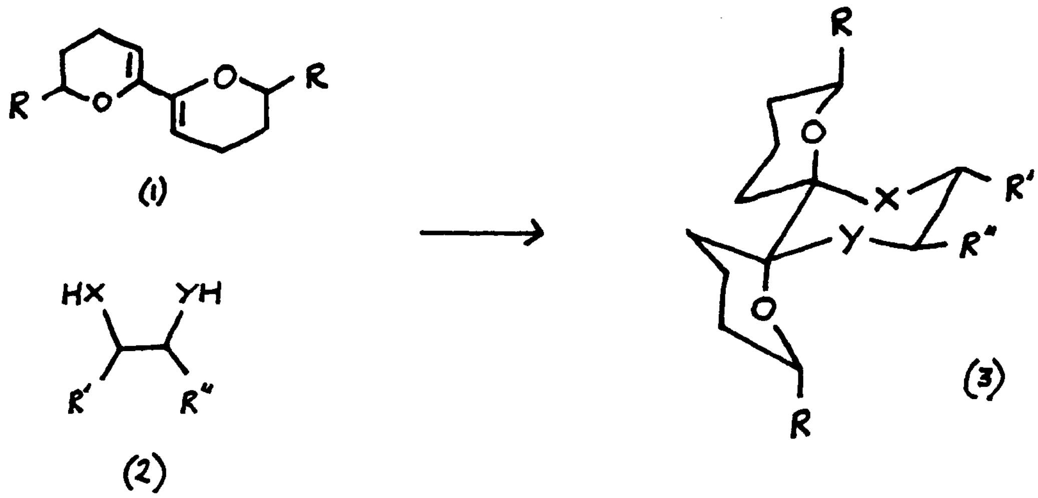


Scheme 1

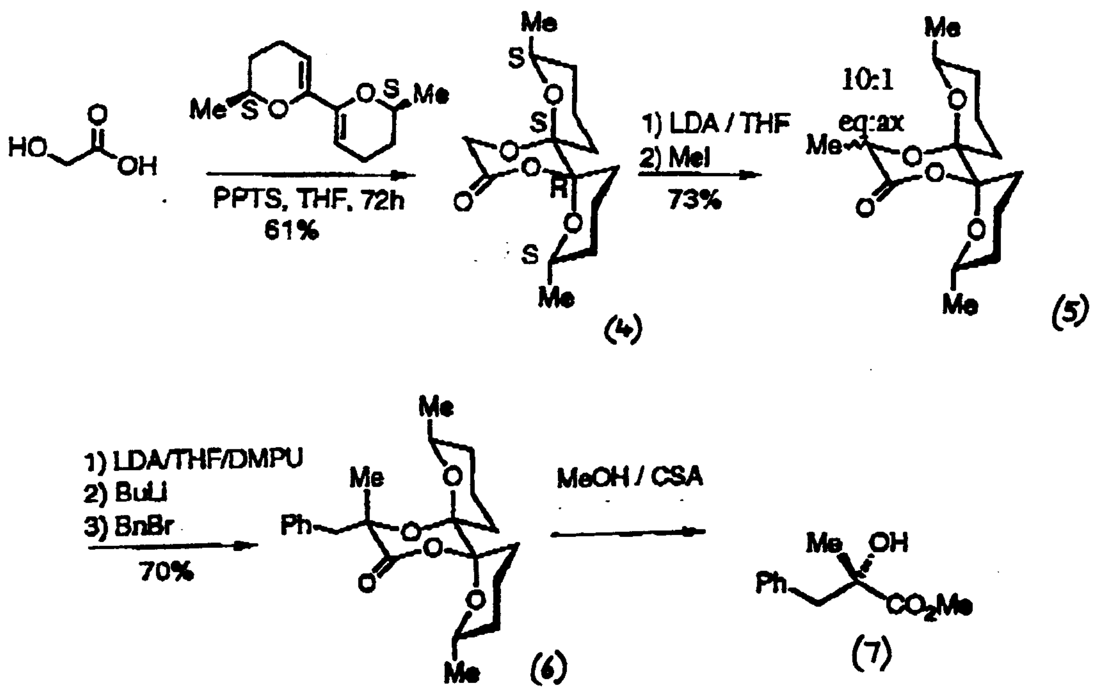


Scheme 3

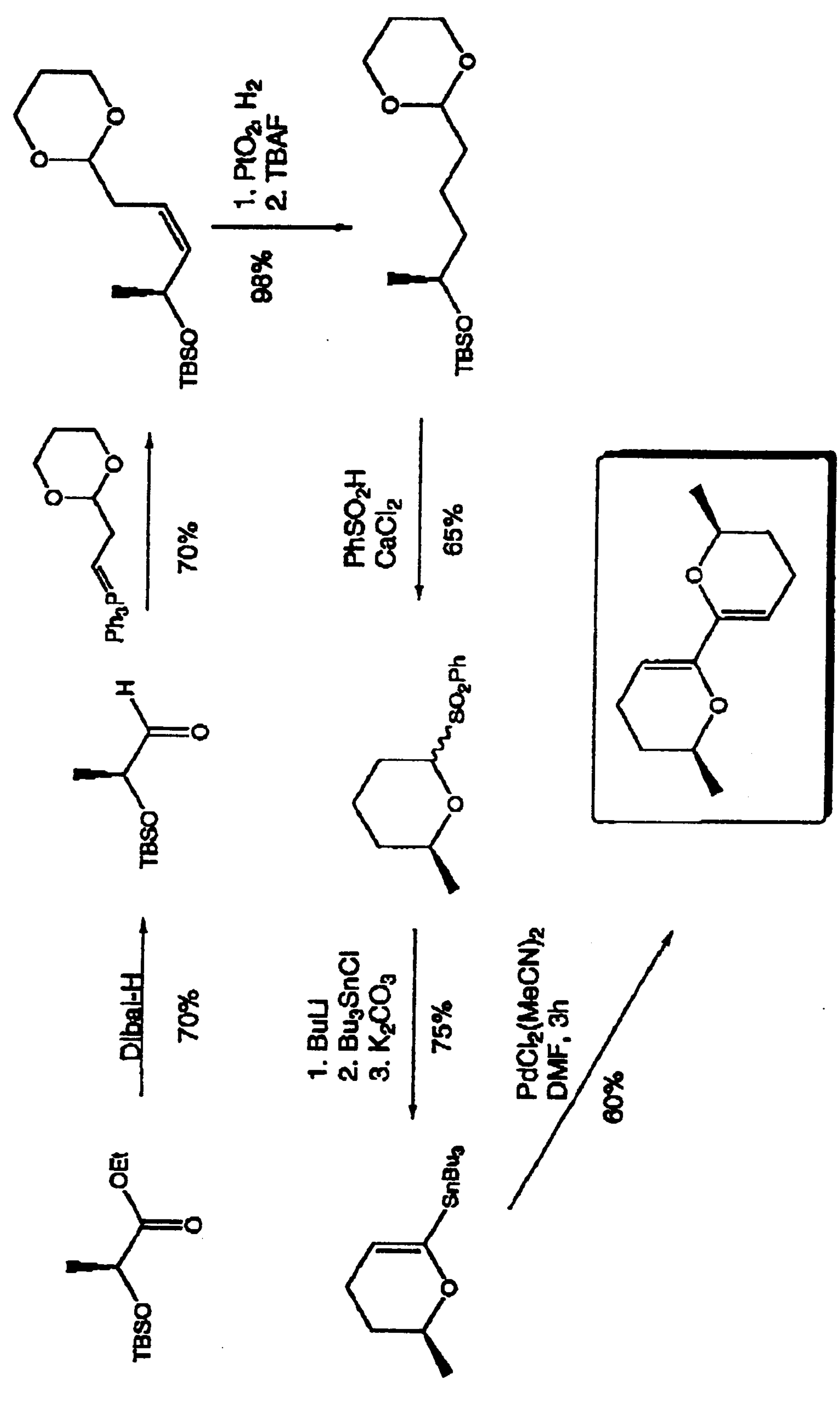
1. PtO2, H2
2. TBAF
98%
70%
Dibal-H
70%
PhSO2H
CaCl2
65%
1. BuLi
2. Bu3SnCl
3. K2CO3
75%
PdCl2(MeCN)2
DMF, 3h
60%

Scheme 2

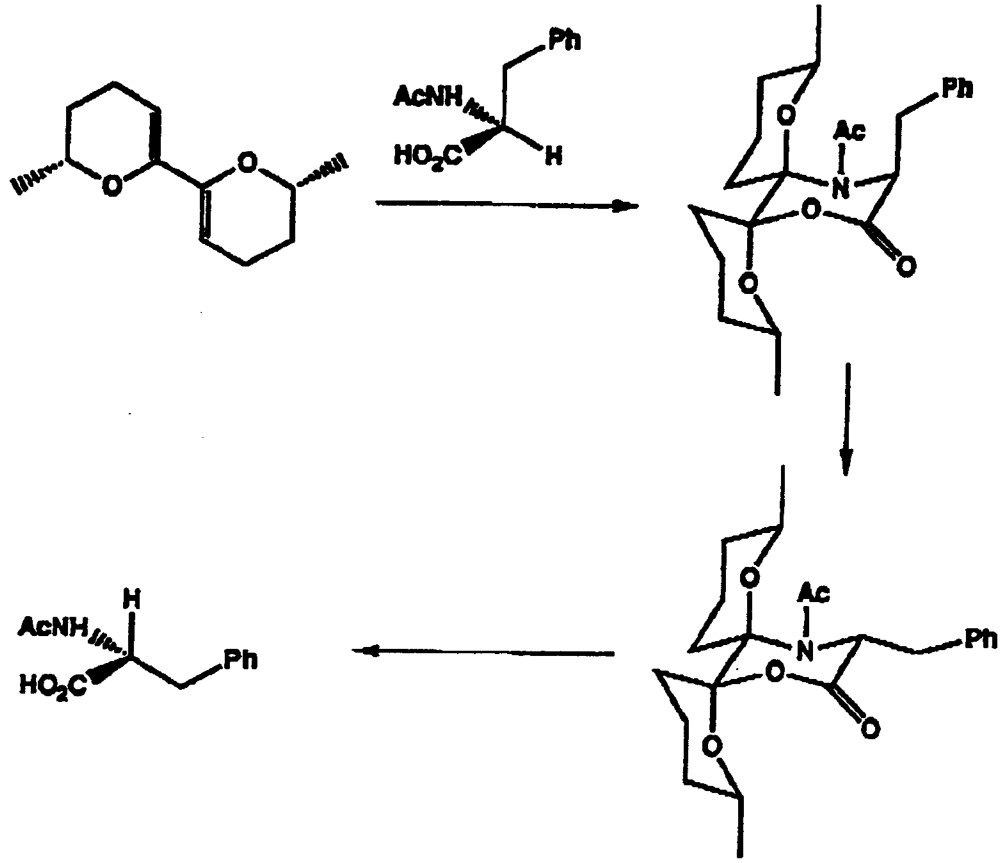

Scheme 4

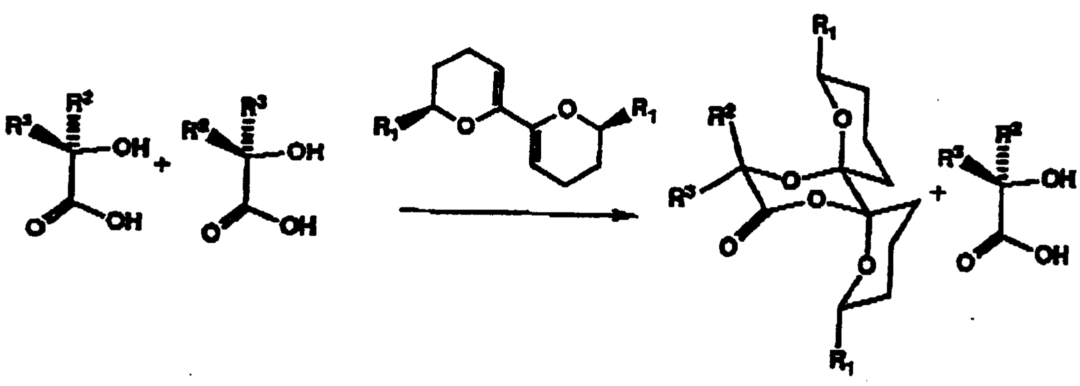

Scheme 5

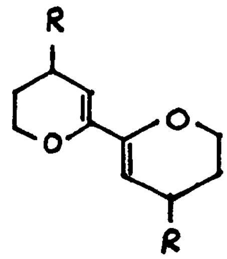

(1A)

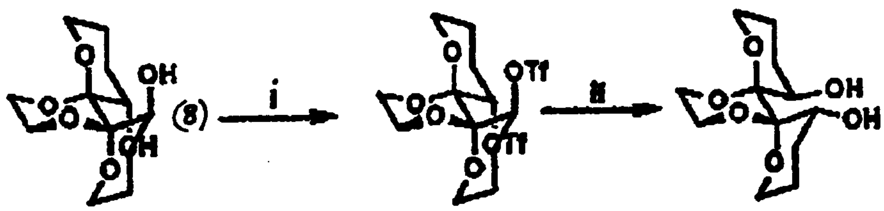


(8)

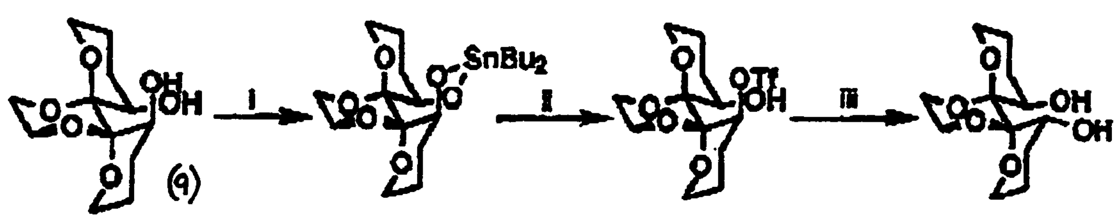


(9)

Scheme 6

## Claims

1. A disubstituted bis-dihydropyran having C2 symmetry, in single enantiomer form.

2. A compound as claimed in claim 1, which is 2,2'-disubstituted.

3. A compound as claimed in claim 1 or claim 2, wherein the substituent is $C_{1-6}$ alkyl or $C_{6-10}$ aryl.

4. A compound as claimed in claim 3, wherein the substituent is methyl or phenyl.

5. A compound as claimed in claim 1, which is 4,4'-disubstituted.

6. A compound as claimed in claim 5, which is a bidentate ligand.

7. A dispiroketal obtainable by reaction of a compound as claimed in any preceding claim with a difunctional compound of the formula R'-CH(XH)-CH(YH)-R".

8. Use of a compound as claimed in any of claims 1 to 6, for the resolution of a chiral difunctional compound as defined in claim 7.

9. Use of a compound as claimed in any of claims 1 to 6, for the disproportionation of an prochiral difunctional compound as defined in claim 7.

10. A process for preparing a chiral difunctional compound as defined in claim 7 in single enantiomer form, which comprises cleaving it from a dispiroketal as claimed in claim 7.

European Patent Office

# PARTIAL EUROPEAN SEARCH REPORT

which under Rule 45 of the European Patent Convention shall be considered, for the purposes of subsequent proceedings, as the European search report

Application Number
EP 94 30 1628

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,X | SYNTHESIS<br>no. 1/2 , 1992 , STUTTGART DE<br>pages 52 - 54<br>S.V. LEY ET AL. 'Dispiroketals in synthesis: preparation of a stable, sterically demanding glyceraldehyde ketal and diastereoselective reactions with simple organometallic reagents'<br>* the whole document, paricularly page 54, left-hand column, lines 2 and 3 *<br>--- | 1-10 | C07D309/16<br>C07D493/20<br>C07D497/20<br>C07B53/00<br>C07B57/00<br>//(C07D493/20, 319:00,311:00, 311:00), (C07D497/20, 327:00,311:00, 311:00) |
| D,A | TETRAHEDRON LETTERS<br>vol. 33, no. 33 , 11 August 1992 , OXFORD GB<br>pages 4767 - 4770<br>S.V. LEY ET AL. 'Dispiroketals in synthesis (part 2): a new group for selective protection of diequatorial vicinal diols in carbohydrates'<br>* the whole document *<br>---<br>-/-- | 1-9 | |
| | | | TECHNICAL FIELDS SEARCHED (Int.Cl.5) |
| | | | C07D |

## INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 18 May 1994 | Allard, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 94 30 1628

| DOCUMENTS CONSIDERED TO BE RELEVANT | | | CLASSIFICATION OF THE APPLICATION (Int.Cl.5) |
|---|---|---|---|
| Category | Citation of document with indication, where appropriate, of relevant passages | Relevant to claim | |
| A | JOURNAL OF THE CHEMICAL SOCIETY, CHEMICAL COMMUNICATIONS<br>no. 17 , 1 September 1990 , LETCHWORTH GB<br>pages 1191 - 1192<br>E. DUBOIS ET AL. 'Formation of C-glycosides by a palladium-catalysed coupling reaction of tributylstannyl glycals with organic halides'<br>* the whole document *<br>--- | 1 | |
| P,X | TETRAHEDRON LETTERS<br>vol. 34, no. 35 , August 1993 , OXFORD GB<br>pages 5649 - 5652<br>G.J. BOONS ET AL. 'Dispiroketals in synthesis (part 4): enantioselective desymmetrization of glycerol using a C2-symmetric disubstituted bis-dihydropyran'<br>* the whole document *<br>----- | 1-10 | |
| | | | TECHNICAL FIELDS SEARCHED (Int.Cl.5) |

EPO FORM 1503 03.82 (P04C10)

EP 94 30 1628

-C-

INCOMPLETE SEARCH

Claims searched incompletely: 1-10

The term "bis-dihydropyran" used in claim 1, without indication of the position of the unsaturation and attachment points of the two rings, is unclear and covers a variety of compounds neither supported by the description nor suited for the use underlying the application. The same lack of clarity applies to claim 7, wherein the symbols R',R",X and Y are undefined. The search has therefore been limited to claims 1-10 as more precisely specified in the description of the application.